# EUROPEAN PATENT APPLICATION

(11) **EP 4 462 116 A1**
(43) Date of publication of application: **13.11.2024**
(21) Application number: 23901754.4
(22) Date of filing: 27.10.2023
(51) Int. Cl.: G01N 33/12, G06Q 50/02, G06T 7/00, G06T 7/11

(54) **FISH-QUALITY DETERMINATION SYSTEM**

(30) Priority: 31.03.2023 JP 2023057275
(71) Applicant: Dentsu Inc., Tokyo 105-7001 (JP)
(72) Inventor: SHIMURA, Kazuhiro, 105-7001 Tokyo (JP)
(74) Representative: Bardehle Pagenberg Partnerschaft mbB Patentanwälte Rechtsanwälte
(86) International application number: PCT/JP2023/038820
(87) International publication number: WO 2024/202161

(57) **Abstract**

A fish quality determination system includes a first machine learning unit that analyzes, by machine learning, a relationship between image data obtained by imaging a cross section of a fish tail and a region of a fish body in the image data, a data acquisition unit that acquires image data of a cross section of a tail of a determination target fish from a user device, a first estimation unit that estimates a region of a body of the determination target fish and outputs an estimation result, using the image data of the cross section of the tail of the determination target fish acquired by the data acquisition unit as an input, based on the relationship analyzed by the first machine learning unit, a generation unit that generates trimming image data in which a region other than the body of the fish is trimmed from the image data of the cross section of the tail of the determination target fish based on the estimation result by the first estimation unit, and a quality determination unit that determines quality of the determination target fish using the trimming image data.

## Description

### Technical Field

The present invention relates to a system for determining quality of a fish (in particular, tuna).

### Background Art

Conventionally, a technique for evaluating freshness of fish and shellfish has been proposed (see, for example, Patent Literature 1.). Conventionally, quality of tuna has been determined by looking at a cross section of a tail of the tuna at a fish wholesale market or the like.

### Citation List

### Patent Literature

Patent Literature 1: JP 2010-286262 A

### Summary of Invention

### Technical Problem

Determining quality of tuna from a cross section of a tail of a fish (in particular, tuna) requires many years of experience, and it has been difficult to determine the quality of tuna without considerable skill. In particular, a cross section of a tail of a fish cut in a non-frozen (raw) state is in a rough state with many irregularities as compared with the cross section of the fish cut in a frozen state, and thus it is more difficult to determine the quality of tuna from the cross section. Therefore, it has been desired to develop a system that allows anyone (not an expert) to easily determine the quality of non-frozen fish.

The present invention has been made in view of the above problems, and an object of the present invention is to provide a fish quality determination system capable of easily determining quality of a fish even by a person who is not an expert.

### Solution to Problem

According to a first aspect, there is provided a fish quality determination system including: a first machine learning unit that analyzes, by machine learning, a relationship between image data obtained by imaging a cross section of a tail of a fish and a region of a body of the fish in the image data; a data acquisition unit that acquires image data of a cross section of a tail of a determination target fish from a user device; a first estimation unit that estimates a region of a body of the determination target fish and outputs an estimation result, using the image data of the cross section of the tail of the determination target fish acquired by the data acquisition unit as an input, based on the relationship analyzed by the first machine learning unit; a generation unit that generates trimming image data in which a region other than the body of the fish is trimmed from the image data of the cross section of the tail of the determination target fish based on the estimation result by the first estimation unit; and a quality determination unit that determines quality of the determination target fish using the trimming image data.

According to this configuration, the region of the body of the fish is estimated using machine learning, and the quality of the determination target fish is determined based on the trimming image data generated based on the estimation result. Therefore, it is possible to accurately determine the quality of the fish by suppressing influence of a region (a region of a vertebra and a background region) other than the region of the body of the fish in the image data of the cross section of the tail of the fish, which becomes noise when determining the quality. As a result, even a person who is not an expert can easily determine the quality of fish.

According to a second aspect, the fish quality determination system according to the first aspect further includes a second machine learning unit that analyzes a relationship between the trimming image data and a region of fat in the trimming image data by machine learning, a second estimation unit that receives the trimming image data as an input, estimates the region of the fat, and outputs an estimation result based on the relationship analyzed by the second machine learning unit, and a fat determination unit that determines a ratio of a region of fat to the trimming image data based on the estimation result by the second estimation unit.

According to this configuration, the region of the fat of the fish is estimated by machine learning using the trimming image data as the input data, and the ratio of the region of the fat in the trimming image data is determined based on the estimation result. Therefore, even when the image data of the cross section of the tail of the fish includes the uneven shape generated when the non-frozen fish is cut, the ratio of the region of the fat can be accurately determined. As a result, even a person who is not an expert can easily determine the quality of fish (ratio of fat).

According to a third aspect, the fish quality determination system according to the first or second aspect further includes a third machine learning unit that analyzes a relationship between the trimming image data and freshness by machine learning, a third estimation unit that estimates the freshness based on the relationship analyzed by the second machine learning unit with the trimming image data as an input and output an estimation result, and a freshness determination unit that determines the freshness based on the estimation result by the third estimation unit.

According to this configuration, the freshness of the fish is estimated by machine learning using the trimming image data as the input data, and the freshness of the fish is determined based on the estimation result. Therefore, the freshness of the fish can be accurately determined even when the image data of the cross section of the tail of the fish includes the uneven shape generated when the non-frozen fish is cut. As a result, even a person who is not an expert can easily determine the quality (freshness) of fish.

According to a fourth aspect, the fish quality determination system according to any one of the first to third aspects further includes a freshness determination unit that determines freshness of the determination target fish using color information of the trimming image data.

According to this configuration, since the freshness of the determination target fish is determined using the color information of the trimming image data, it is possible to accurately determine the freshness of the fish even when the image data of the cross section of the tail of the fish includes the uneven shape generated when the non-frozen fish is cut. As a result, even a person who is not an expert can easily determine the quality (freshness) of fish.

According to a fifth aspect, in the fish quality determination system according to the third or fourth aspect, the quality determination unit determines quality of the determination target fish based on a ratio of fat determined by the fat determination unit and freshness determined by the freshness determination unit.

According to this configuration, the quality of the determination target fish can be determined based on the ratio of the fat and the freshness. As a result, even a person who is not an expert can easily determine the quality of fish.

According to a sixth aspect, there is provided a method performed in a fish quality determination system, the method including: a first machine learning step of analyzing, by machine learning, a relationship between image data obtained by imaging a cross section of a tail of a fish and a region of a body of the fish in the image data; a data acquisition step of acquiring image data of a cross section of a tail of a determination target fish from a user device; a first estimation step of estimating a region of a body of the determination target fish and outputting an estimation result, using the image data of the cross section of the tail of the determination target fish acquired in the data acquisition step as an input, based on the relationship analyzed in the first machine learning step; a generation step of generating trimming image data in which a region other than the body of the fish is trimmed from image data of the cross section of the tail of the determination target fish based on the estimation result by the first estimation step; and a quality determination step of determining quality of the determination target fish using the trimming image data.

Also by this method, similarly to the above system, the region of the body of the fish is estimated using machine learning, and the quality of the determination target fish is determined based on the trimming image data generated based on the estimation result. Therefore, it is possible to accurately determine the quality of the fish by suppressing influence of a region (a region of a vertebra and a background region) other than the region of the body of the fish in the image data of the cross section of the tail of the fish, which becomes noise when determining the quality. As a result, even a person who is not an expert can easily determine the quality of fish.

According to a seventh aspect, there is provided a program for causing a computer to execute the method according to the sixth aspect.

Also with this program, similarly to the above system, the region of the body of the fish is estimated using machine learning, and the quality of the determination target fish is determined based on the trimming image data generated based on the estimation result. Therefore, it is possible to accurately determine the quality of the fish by suppressing influence of a region (a region of a vertebra and a background region) other than the region of the body of the fish in the image data of the cross section of the tail of the fish, which becomes noise when determining the quality. As a result, even a person who is not an expert can easily determine the quality of fish.

According to an eighth aspect, there is provided a computer-readable storage medium storing the program according to the seventh aspect.

### Brief Description of Drawings

Fig. 1 is a block diagram of a quality determination system according to one embodiment of the present invention.
Fig. 2 is a flowchart illustrating an example of processing of the fish quality determination system according to one embodiment of the present invention.
Fig.3 is an example of image data of a cross section of a tail of a non-frozen fish (tuna) according to one embodiment of the present invention.
Fig. 4 is an example of trimming image data in one embodiment of the present invention.

### Description of Embodiments

Hereinafter, a fish quality determination system according to one embodiment of the present invention will be described with reference to the drawings. In the present embodiment, a case of a quality determination system for tuna will be exemplified.

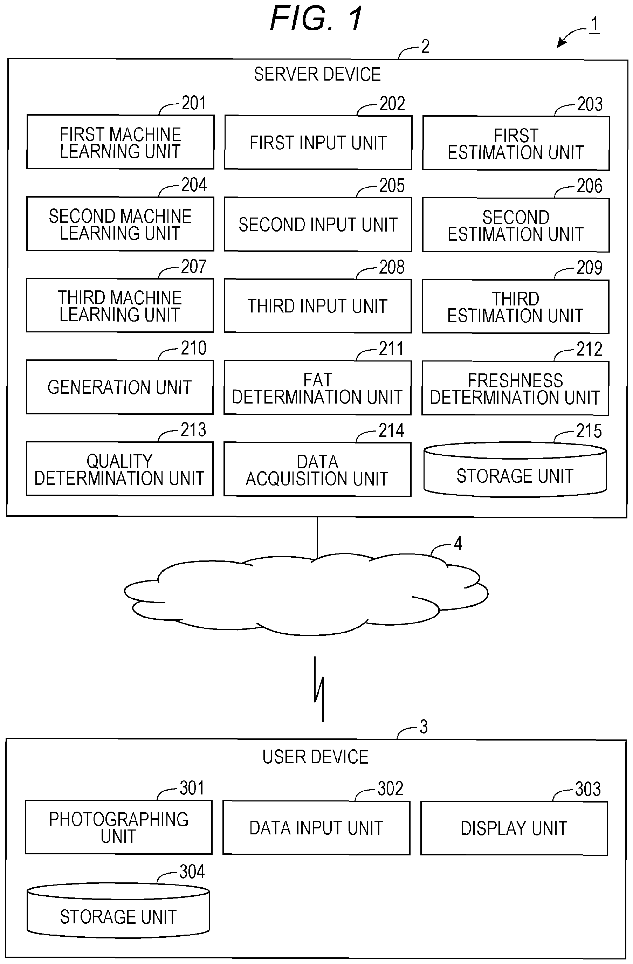

A configuration of a quality determination system according to one embodiment of the present invention will be described with reference to the drawings. Fig. 1 is a block diagram illustrating a configuration of a quality determination system of the present embodiment. As illustrated in Fig. 1, a quality determination system 1 includes a server device 2 and a user device 3. The server device 2 and the user device 3 are communicably connected to each other via a network 4. For example, the server device 2 is a cloud server or the like owned by a provider of a quality determination service, and the user device 3 is a smartphone or the like owned by a user of the quality determination service.

As illustrated in Fig. 1, the server device 2 includes a first machine learning unit 201, a first input unit 202, a first estimation unit 203, a second machine learning unit 204, a second input unit 205, a second estimation unit 206, a third machine learning unit 207, a third input unit 208, a third estimation unit 209, a generation unit 210, a fat determination unit 211, a freshness determination unit 212, a quality determination unit 213, a data acquisition unit 214, and a storage unit 215.

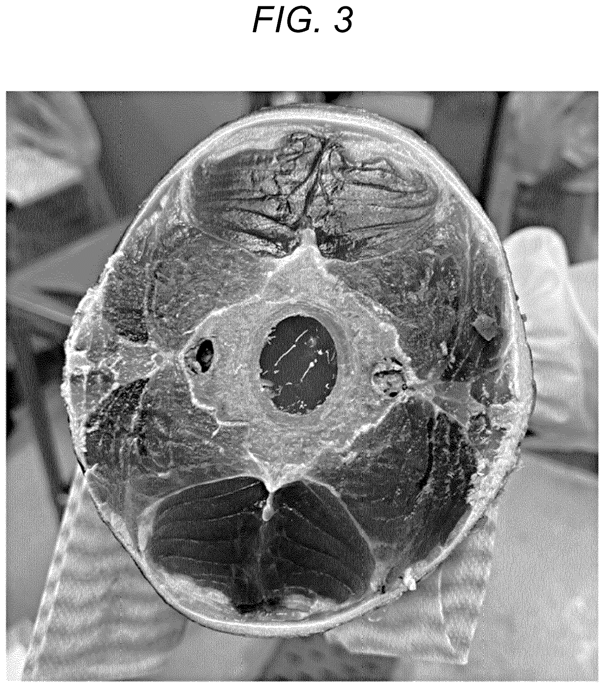

The first machine learning unit 201 analyzes a relationship between image data (see Fig. 3) obtained by imaging a cross section of a tail of a fish and a region of a body of the fish in the image data by machine learning. For this machine learning, an arbitrary method such as deep learning by a neural network is used.

For example, in the case of the neural network, the image data of the cross section of the tail of the tuna is input to an input layer, and the data regarding the region of the body in the image data is output from an output layer. Then, weighting coefficients between neurons of the neural network are optimized by supervised learning using analysis data (teacher data) in which data input to the input layer and data output from the output layer are associated with each other. As the teacher data of the data related to the region of the body in the image data of the cross section of the tail of the tuna, for example, data in which an expert labels the image data of the cross section of the tail of the tuna for each region is used.

Image data of a cross section of a tail of a determination target tuna is input to the first input unit 202. The image data of the cross section of the tail of the determination target tuna is acquired, for example, by imaging the cross section of the tail of the tuna at fishing factories in various places.

Based on the relationship analyzed by the first machine learning unit 201, the first estimation unit 203 receives the image data of the cross section of the tail of the determination target tuna input by the first input unit 202, and estimates and outputs data related to the region of the body in the image data of the cross section of the tail of the determination target tuna. For example, in the case of the above neural network, the image data of the cross section of the tail of the determination target tuna is input to the input layer, the data regarding the region of the body in the image data of the tail of the determination target tuna is estimated, and the output (estimation result) is output from the output layer, whereby the data regarding the region of the body in the image data of the cross section of the tail of the determination target tuna is estimated.

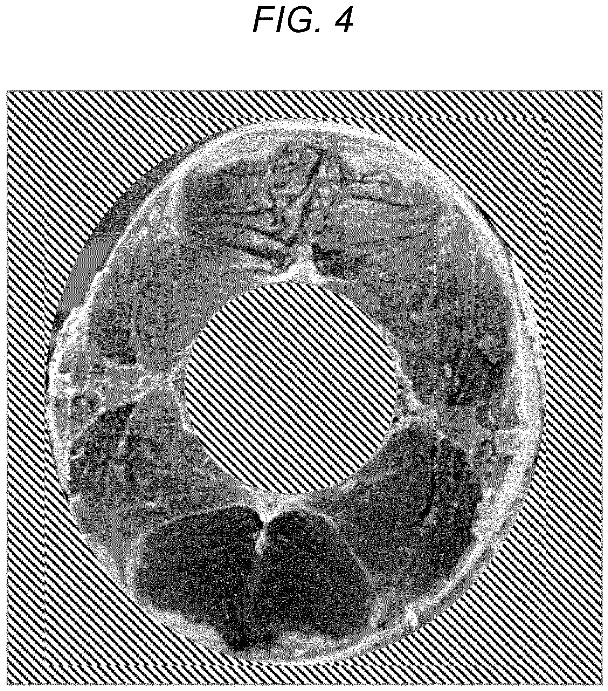

The second machine learning unit 204 analyzes, by machine learning, a relationship between trimming image data (see Fig. 4) generated by the generation unit 210 to be described later and a region of fat in the trimming image data. For this machine learning, an arbitrary method such as deep learning by a neural network is used.

For example, in the case of the neural network, the trimming image data is input to the input layer, and the data related to the region of the fat in the image data is output from the output layer. Then, weighting coefficients between neurons of the neural network are optimized by supervised learning using analysis data (teacher data) in which data input to the input layer and data output from the output layer are associated with each other. As the teacher data of data related to the region of the fat in the trimming image data, for example, data in which an expert labels the trimming image data for each region is used.

The trimming image data of the determination target tuna is input to the second input unit 205. The trimming image data of the determination target tuna is generated by the generation unit 210 to be described later.

Based on the relationship analyzed by the second machine learning unit 204, the second estimation unit 206 receives the trimming image data of the determination target tuna input by the second input unit 205 as input, and estimates and outputs data related to the region of the fat in the trimming image data. For example, in the above neural network, the trimming image data of the determination target tuna is input to the input layer, the data on the region of the fat in the trimming image data of the determination target tuna is estimated, and the output (estimation result) is output from the output layer, whereby the data on the region of the fat in the trimming image data of the determination target tuna is estimated.

The third machine learning unit 207 analyzes a relationship between the trimming image data (see Fig. 4) generated by the generation unit 210 to be described later and the freshness of the fish by the machine learning. For this machine learning, an arbitrary method such as deep learning by a neural network is used.

For example, in the case of a neural network, the trimming image data is input to an input layer, and data regarding freshness corresponding to the trimming image data is output from an output layer. Then, weighting coefficients between neurons of the neural network are optimized by supervised learning using analysis data (teacher data) in which data input to the input layer and data output from the output layer are associated with each other. As teacher data of data regarding freshness corresponding to the trimming image data, for example, data obtained by attaching a freshness label (for example, ranks of 1 to 5 (1 represents lowest freshness, and 5 represents highest freshness)) to the trimming image data by an expert is used.

The trimming image data of the determination target tuna is input to the third input unit 208. The trimming image data of the determination target tuna is generated by the generation unit 210 to be described later.

Based on the relationship analyzed by the third machine learning unit 207, the third estimation unit 209 receives, as input, the trimming image data of the determination target tuna input by the third input unit 208, and estimates and outputs data related to freshness corresponding to the trimming image data. For example, in the case of the above neural network, the trimming image data of the determination target tuna is input to the input layer, the data regarding freshness corresponding to the trimming image data of the determination target tuna is estimated, and the output (estimation result) is output from the output layer, whereby the data regarding freshness corresponding to the trimming image data of the determination target tuna is estimated.

The generation unit 210 generates, based on the estimation result (data regarding the region of the body of the fish in the image data of the cross section of the tail of the fish) by the first estimation unit 203, trimming image data obtained by trimming the region (the region of the vertebra and the background region) other than the fish body from the image data of the cross section of the tail of the determination target fish. Fig. 4 is a view illustrating an example of trimming image data generated by generation unit 210. As illustrated in Fig. 4, the generation unit 210 generates, from the image data of the cross section of the tail of the fish, trimming image data obtained by trimming the region of the vertebra located in the central portion of the cross section and the background region. The trimming image data generated by the generation unit 210 is input to the second input unit 205 and the third input unit 208.

The fat determination unit 211 determines the ratio (content rate) of the region of the fat to the trimming image data based on the estimation result (data related to the region of the fat in the trimming image data) by the second estimation unit 206. For example, the ratio of the area of the region of the fat to the total area of the trimming image data (image data of the region of the body of the fish) may be set as the content rate of the fat.

The freshness determination unit 212 determines the freshness of the determination target fish based on an estimation result (freshness of fish corresponding to trimming image data) by the third estimation unit 209. Here, the freshness determination unit 212 may adopt freshness estimated by third estimation unit 209 as it is, or may determine a value obtained by normalizing a numerical value output by third estimation unit 209 to a predetermined numerical range as the freshness.

Note that the freshness determination unit 212 may determine the freshness from the HSV color information of the trimming image data without using the estimation result of the third estimation unit 209. For example, since the color of the cross section of the body of the fish becomes more vivid red as the freshness is higher, the freshness determination unit 212 may extract red (Hue: 0 to 60 degrees and 300 to 360 degrees) from the trimming image data, obtain saturation (0 to 100%) of the extracted red, and then determine a value obtained by normalizing the obtained saturation to a predetermined numerical range (For example, 1 to 5) as the freshness.

The quality determination unit 213 determines the quality of the determination target fish based on the ratio (content rate) of the fat determined by the fat determination unit 211 and the freshness determined by the freshness determination unit 212. For example, the fresher and fatty the tuna, the higher the value, so the quality is determined in such a way that the fresher and fatty the tuna, the higher the quality.

The data acquisition unit 214 acquires image data of the cross section of the tail of the determination target tuna from the user device 3.

The storage unit 215 stores the image data acquired from the user device 3 and the trimming image data generated by the generation unit 210. In addition, the storage unit 215 stores the estimation results output from the first estimation unit 203, the second estimation unit 206, and the third estimation unit 209.

Next, the user device 3 will be described. As illustrated in Fig. 1, the user device 3 includes a imaging unit 301, a data input unit 302, a display unit 303, and a storage unit 304.

The imaging unit 301 is realized by the camera function of the user device 3, and generates image data of the cross section of the tail of the tuna by imaging the cross section of the tail of the tuna (see Fig. 3). The generated image data of the cross section of the tail of the tuna may be associated with data of the imaging date.

The data input unit 302 has a function of inputting various data. Information (for example, identification information, weight, fishing area, fishing boat name, and the like of the determination target fish) on determination target tuna is input from the data input unit 302.

The display unit 303 has a function of displaying various data. The display unit 303 displays at least one of the ratio (content rate) of fat determined by the fat determination unit 211, the freshness determined by the freshness determination unit 212, and the quality of the fish determined by the quality determination unit 213. When the display unit 303 has a touch panel function, the display unit 303 can also serve as the data input unit 302.

The storage unit 304 stores the image data of the tuna photographed by the imaging unit 301 and information input via the data input unit 302. In addition, the storage unit 304 stores data regarding the ratio (content rate) of the fat, the freshness, and the quality of the fish output from the server device 2.

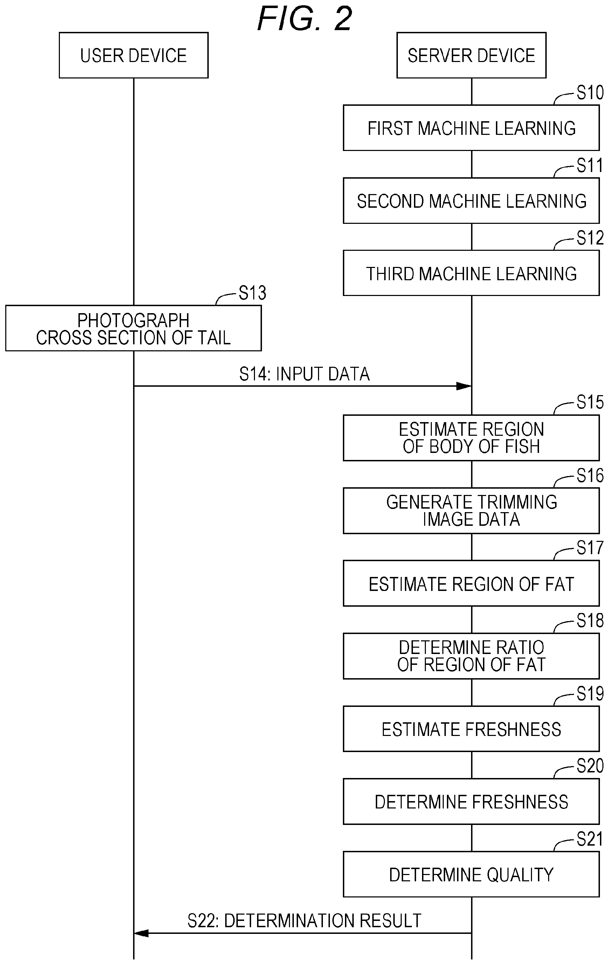

An operation of the quality determination system 1 configured as described above will be described with reference to the sequence diagram of Fig. 2.

As illustrated in Fig. 2, in the quality determination system 1 of the present embodiment, first, the first machine learning unit 201 of the server device 2 analyzes the relationship between the image data of the cross section of the tail of the tuna and the region of the body in the image data in advance by machine learning (S10).

The second machine learning unit 204 of the server device 2 analyzes the relationship between the trimming image data and the region of the fat in the trimming image data in advance by machine learning (S11).

In addition, the third machine learning unit 207 of the server device 2 analyzes the relationship between the trimming image data and the freshness corresponding to the trimming image data in advance by machine learning (S12).

When the image of the cross section of the tail of the tuna is captured by the user device 3 (S13), the user device 3 transmits image data of the cross section of the tail of the tuna to the server device 2 (S14).

Upon receiving the image data of the cross section of the tail of the tuna from the user device 3, the server device 2 estimates the region of the body of the tuna by the first estimation unit 203 (S15).

Then, the server device 2 causes the generation unit 210 to generate trimming image data by using the estimation result (data regarding the region of the body of the tuna) estimated by the first estimation unit 203 (S16).

Next, the server device 2 estimates the region of the fat by the second estimation unit 206 using the trimming image data generated in Step S16 as input data (S17).

Then, the server device 2 causes the fat determination unit 211 to determine the ratio (content rate) of the region of the fat of the tuna by using the estimation result (data related to the fat region in the trimming image data) estimated by the second estimation unit 206 (S18) .

Next, the server device 2 uses the trimming image data generated in Step S16 as input data, and estimates the freshness of the determination target tuna by the third estimation unit 209 (S19).

Then, the server device 2 causes the freshness determination unit 212 to determine the freshness of the determination target tuna by using the estimation result estimated by the third estimation unit 209 or the color information of the trimming image data (S20).

In addition, the server device 2 causes the quality determination unit 213 to determine the quality of the determination target tuna based on the ratio (content rate) of the fat determined by the fat determination unit 211 and the freshness determined by the freshness determination unit 212 (S21) .

Then, the server device 2 transmits the determination result (at least one of the ratio of the fat, the freshness, and the quality) to the user device 3.

According to the quality determination system 1 of the present embodiment, the quality determination system 1 includes the first machine learning unit 201 that analyzes, by machine learning, the relationship between the image data obtained by imaging the cross section of the tail of the fish and the region of the body of the fish in the image data, the data acquisition unit 214 that acquires the image data of the cross section of the tail of the determination target fish from the user device, the first estimation unit 203 that estimates the region of the body of the determination target fish and outputs the estimation result, using the image data of the cross section of the tail of the determination target fish acquired by the data acquisition unit 214 as the input, based on the relationship analyzed by the first machine learning unit, the generation unit 210 that generates the trimming image data in which a region other than the body of the fish is trimmed from the image data of the cross section of the tail of the determination target fish based on the estimation result by the first estimation unit 203, and the quality determination unit 213 that determines the quality of the determination target fish using the trimming image data. Accordingly, it is possible to accurately determine the quality of the fish by suppressing influence of a region (region of a vertebra and a background region) other than the region of the body of the fish in the image data of the cross section of the tail of the fish, which becomes noise when determining the quality. As a result, even a person who is not an expert can easily determine the quality of fish.

According to the quality determination system 1 of the present embodiment, machine learning is used for the determination of the ratio (content rate) of the fat and the determination of the freshness. Therefore, even when the image data of the cross section of the tail of the fish includes the uneven shape generated when a non-frozen fish is cut, the ratio (content rate) of the fat and the freshness can be accurately determined, and as a result, the quality of the fish can be accurately determined. As a result, even a person who is not an expert can more easily determine the quality of the fish.

Although the present embodiment has been described above, any part or all of the functional units described in the present specification may be realized by a program.

The program referred to in the present specification may be distributed by being non-temporarily recorded in a computer-readable recording medium, may be distributed via a communication line (including wireless communication) such as the Internet, or may be distributed in a state of being installed in an arbitrary terminal.

Based on the above description, a person skilled in the art may be able to conceive additional effects and various modifications of the present invention, but aspects of the present invention are not limited to the individual embodiments described above. Various additions, modifications, and partial deletions can be made without departing from the conceptual idea and spirit of the present invention derived from the contents defined in the claims and equivalents thereof.

For example, what is described herein as a single device (alternatively, a single member, and the same applies hereinafter) (including what is depicted in the drawings as a single device) may be implemented by a plurality of devices. Conversely, what is described herein as a plurality of devices (including what is depicted in the drawings as a plurality of devices) may be realized by one device. Alternatively, some or all of means or functions assumed to be included in a certain device (for example, a server) may be included in another device (for example, a user terminal).

In addition, not all the matters described in the present specification are essential requirements. In particular, matters described in the present specification and not described in the claims can be regarded as any additional matters.

It should be noted that the applicant of the present invention is merely aware of the invention disclosed in the literature in the column of "Citation List" in the present specification, and the present invention is not necessarily intended to solve the problem in the invention disclosed in the literature. The technical problem should be recognized in consideration of the entire specification. For example, in the present specification, in a case where there is a description that a predetermined effect is exhibited by a specific configuration, it can be said that the problem of reversing the predetermined effect is solved. However, such a specific configuration is not necessarily an essential requirement.

### Industrial applicability

As described above, the fish quality determination system according to the present invention has an effect that quality of fish can be easily determined even by a person who is not an expert, and is useful as a tuna quality determination system or the like.

## Claims

1. A fish quality determination system comprising:
a first machine learning unit that analyzes, by machine learning, a relationship between image data obtained by imaging a cross section of a tail of a fish and a region of a body of the fish in the image data;
a data acquisition unit that acquires image data of a cross section of a tail of a determination target fish from a user device;
a first estimation unit that estimates a region of a body of the determination target fish and outputs an estimation result, using the image data of the cross section of the tail of the determination target fish acquired by the data acquisition unit as an input, based on the relationship analyzed by the first machine learning unit;
a generation unit that generates trimming image data in which a region other than the body of the fish is trimmed from the image data of the cross section of the tail of the determination target fish based on the estimation result by the first estimation unit; and
a quality determination unit that determines quality of the determination target fish using the trimming image data.

2. The fish quality determination system according to claim 1, further comprising:
a second machine learning unit that analyzes a relationship between the trimming image data and a region of fat in the trimming image data by machine learning;
a second estimation unit that receives the trimming image data as an input, estimates the region of the fat, and outputs an estimation result based on the relationship analyzed by the second machine learning unit; and
a fat determination unit that determines a ratio of a region of fat to the trimming image data based on the estimation result by the second estimation unit.

3. The fish quality determination system according to claim 2, further comprising:
a third machine learning unit that analyzes a relationship between the trimming image data and freshness by machine learning;
a third estimation unit that estimates the freshness based on the relationship analyzed by the second machine learning unit with the trimming image data as an input and output an estimation result; and
a freshness determination unit that determines the freshness based on the estimation result by the third estimation unit.

4. The fish quality determination system according to claim 2, further comprising a freshness determination unit that determines freshness of the determination target fish using color information of the trimming image data.

5. The fish quality determination system according to claim 3 or 4, wherein the quality determination unit determines quality of the determination target fish based on a ratio of fat determined by the fat determination unit and freshness determined by the freshness determination unit.

6. A method performed in a fish quality determination system, the method comprising:
a first machine learning step of analyzing, by machine learning, a relationship between image data obtained by imaging a cross section of a tail of a fish and a region of a body of the fish in the image data;
a data acquisition step of acquiring image data of a cross section of a tail of a determination target fish from a user device;
a first estimation step of estimating a region of a body of the determination target fish and outputting an estimation result, using the image data of the cross section of the tail of the determination target fish acquired in the data acquisition step as an input, based on the relationship analyzed in the first machine learning step;
a generation step of generating trimming image data in which a region other than the body of the fish is trimmed from image data of the cross section of the tail of the determination target fish based on the estimation result by the first estimation step; and
a quality determination step of determining quality of the determination target fish using the trimming image data.

7. A program for causing a computer to execute the method according to claim 6.

8. A computer-readable storage medium storing the program according to claim 7.
